# EUROPÄISCHE PATENTANMELDUNG

(11) **EP 1 195 091 A1**
(43) Veröffentlichungstag der Anmeldung: **10.04.2002**
(21) Anmeldenummer: 01119287.9
(22) Anmeldetag: 10.08.2001
(51) Int. Cl.: A01N 47/28, A01N 33/12, A01N 33/08, A01N 33/04, A61L 2/18

(54) **Desinfektionsmittelkonzentrate sowie die Verwendung derselben zur maschinellen Instrumentendesinfektion**

(30) Priorität: 19.08.2000 DE 10040664
(71) Anmelder: Bode Chemie GmbH & Co., D-22525 Hamburg (DE)
(72) Erfinder: Fehling, Thomas, 22305 Hamburg (DE); Knieler, Roland, Dr., 21218 Harmstorf (DE)
(74) Vertreter: Hansert, Reiner M.

(57) **Zusammenfassung**

Desinfektionsmittelformulierungen, welche neben einem Gehalt an Wirkstoffen gewählt aus den Gruppen quatemäre Ammoniumverbindungen, Alkylamine, Alkylthiouroniumsalze, α, ω-Alkylendithiouroniumsalze und/oder Gemische derselben
a) 1 bis 50 Gew.-% mindestens einer Komplexbildnerkomponente,
b) 1 bis 50 Gew.-% mindestens eines nichtionischen Tensids und
c) 0,1 bis 10 Gew.-% mindestens eines hydrophoben Schaumregulators enthalten,
wobei die Prozentangaben jeweils auf das Gesamtgewicht der Formulierungen bezogen sind.

## Beschreibung

Die vorliegende Erfindung betrifft Desinfektionsmittelkonzentrate und ihre Verwendung zur maschinellen Instrumentendesinfektion.

Als Desinfektionsmittel zur Desinfektion von Instrumenten und Wäsche sind eine Vielzahl mikrobizid wirksamer chemischer Substanzen bzw. Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so daß man üblicherweise eine ausreichende Desinfektion durch geeignete Wirkstoffkombinationen erzielen kann.

Der Stand der Technik kennt zur Desinfektion von medizinisch-chirurgischen Instrumenten eine Reihe von Wirkstoffen, insbesondere Aldehyde, wie beispielsweise Formaldehyd oder Glutaraldehyd, quaternäre Ammoniumverbindungen und langkettige Amine sowie Phenole. Allerdings weisen die genannten Verbindungen einige Nachteile auf:

Aldehyde fixieren Reste von Blut und Eiweiß durch chemische Reaktion an den zu desinfizierenden Gegenständen, so daß diese nach der Desinfektion schwer zu reinigen sind.

Quaternäre Ammoniumverbindungen und langkettige Amine werden häufig in der manuellen Instrumentendesinfektion verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quatemären Ammoniumverbindungen allerdings nicht geeignet, weil es infolge der Turbulenzen in der Reinigungsmaschine zu einer starken, unerwünschten Schaumbildung kommt. Ein weiterer entscheidender Nachteil ist das eingeengte Wirkungsspektrum quaternärer Ammoniumverbindungen, die nicht gegen gramnegative Bakterien wirken.

Auch Alkylamine, wie beispielsweise N,N-Bis-(3-aminopropyl)-laurylamin, sind prinzipiell zur Instrumentendesinfektion geeignet. Allerdings haben auch sie den Nachteil, in einer Reinigungsmaschine stark zu schäumen, weshalb auch sie sich für die chemo-thermische Desinfektion bislang nicht durchgesetzt haben.

Ferner weisen auch Thiouroniumsalze (Alkylthiouroniumsalze, α, ω-Alkylendithiouroniumsalze) aufgrund ihres aliphatischen Molekülteils einen ausgeprägten Tensidcharakter auf, der eine hohe Reinigungswirkung erwarten läßt. Damit verbunden ist allerdings auch eine starke Schaumbildung, so daß Thiouroniumsalzlösungen für die maschinelle Instrumentendesinfektion bisher nicht eingesetzt werden konnten.

Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Aufgabe der Erfindung war es daher, Desinfektionsmittel zur maschinellen Instrumentendesinfektion zu finden, welche die Nachteile des Standes der Technik nicht aufweisen, welche also eine gute Reinigungsleistung bei zugleich geringer Toxizität zeigen, weder Eiweiß noch Blut an den zu desinfizierenden Gegenständen fixieren, keinen unangenehmen Geruch haben und insbesondere in Wasch- und Desinfektionsautomaten für chirurgische Instrumente keine störende Schaumbildung verursachen. Ferner sollte das Desinfektionsmittel möglichst aldehydfrei sein.

Zwar ließe sich durch den Einsatz von Entschäumern - z. B. auf Silikonbasis - die Schaumentwicklung der tensidischen Wirkstoffe (quatemäre Ammoniumverbindungen oder Alkylamine) dämpfen. Allerdings wären hierzu für dieses Einsatzgebiet so große Mengen notwendig, daß dies keine Lösung des Problems darstellt. Derartige Entschäumer lassen sich innerhalb eines chemo-thermischen Desinfektionsprogrammes nur schwer wieder von den Instrumenten und dem Maschineninnenraum entfernen und können zudem zu Materialverträglichkeitsproblemen mit den empfindlichen thermolabilen Instrumenten und darüber hinaus zu einer Beeinträchtigung der Wirksamkeit führen.

Auf der anderen Seite besteht aber ein großer Bedarf an Desinfektionsmitteln für die chemo-thermische Instrumentendesinfektion, die einerseits die Vorteile aldehydfreier Wirkstoffe aufweisen, aber andererseits deren Nachteile nicht zeigen.

Es war überraschend und für den Fachmann nicht vorauszusehen, daß
Desinfektionsmittelformulierungen, welche neben einem Gehalt an Wirkstoffen gewählt aus den Gruppen quatemäre Ammoniumverbindungen, Alkylamine, Alkylthiouroniumsalze, α, ω-Alkylendithiouroniumsalze und/oder Gemische derselben
a) 1 bis 50 Gew.-% mindestens einer Komplexbildnerkomponente,
b) 1 bis 50 Gew.-% mindestens eines nichtionischen Tensids und
c) 0,1 bis 10 Gew.-% mindestens eines hydrophoben Schaumregulators enthalten,
wobei die Prozentangaben jeweils auf das Gesamtgewicht der Formulierungen bezogen sind,
den geschilderten Nachteilen des Standes der Technik Abhilfe schaffen würden.

Die erfindungsgemäßen Desinfektionsmittelformulierungen (in folgenden auch "Desinfektionsmittelkonzentrate" genannt) werden entweder als solche, besonders vorteilhaft aber nach Verdünnen mit Wasser - in Form einer sogenannten "Gebrauchslösung" - als Desinfektionsmittel verwendet.

Desinfektionsmittelformulierungen im Sinne der vorliegenden Erfindung sind besonders geeignet zum Desinfizieren von Instrumenten, insbesondere zur maschinellen Instrumentenaufbereitung.

Die bakterizide und fungizide Wirkung der erfindungsgemäßen Formulierungen ist ausgesprochen gut. Von Vorteil für ihre Anwendung im Bereich der maschinellen Instrumentenaufbereitung ist insbesondere ihre inaktivierende Wirkung auf das Hepatitis B-Virus. Das Hepatitis B-Oberflächenantigen wird bereits nach einer Einwirkungszeit von 5 Minuten bei der maschinellen Anwendung zerstört.

Überraschenderweise bilden die erfindungsgemäßen Formulierungen - insbesondere in Form einer Gebrauchslösung - im Gegensatz zum Verhalten der Einzelwirkstoffe selbst bei starker Turbulenz nur eine sehr geringe Schaummenge, wodurch ein Einsatz dieser Wirkstoffe für die maschinelle chemo-thermische Instrumentendesinfektion möglich wird, ohne daß Silikonentschäumer oder größere Mengen anderer stark hydrophober Schaumdämpfer, wie z. B. Alkylpolyglygolether, eingesetzt werden müssen.

Femer verursachen Desinfektionsmittelformulierungen gemäß der vorliegenden Erfindung in ihrer Umgebung keine bzw. nur eine geringe Korrosion. Dies ist naturgemäß insbesondere für die Instrumentendesinfektion von Vorteil, da sowohl die zu desinfizierenden Gegenstände als auch die verwendeten Wasch- und Desinfektionsautomaten im allgemeinen aus Metall gefertigt sind bzw. Metallteile enthalten.

Die Desinfektionsmittelkonzentrate stellen klare, einphasige Mischungen dar, welche in einem Temperaturbereich von 10 bis 50 °C völlig stabil sind. Die wäßrige Gebrauchslösung hingegen, welche vorteilhaft beispielsweise 0,2 bis 2,0 Gew.-% an Konzentrat enthält, stellt in einem Temperaturbereich von 20 bis 60 °C eine trübe Emulsion dar, die äußerst schaumarm und somit ganz besonders für den maschinellen Einsatzbereich geeignet ist.

Es war in keiner Weise vorhersehbar, daß die Kombination der tensidischen Wirkstoffe mit den erfindungsgemäßen Hilfsstoffen, die üblicherweise keine schaumdämpfenden Eigenschaften haben und vollständig wasserlöslich sind, den oben beschriebenen Effekt ermöglichen würden. Ferner war insbesondere erstaunlich, daß die erfindungsgemäßen Desinfektionsmittelformulierungen ohne den Zusatz von Lösungsvermittlern stark hydrophobe Schaumregulatoren enthalten können, welche üblicherweise selbst in geringsten Mengen nicht wasserlöslich sind.

Erfindungsgemäß vorteilhafte Komplexbildnerkomponenten sind Nitriloessigsäure und ihre Salze, insbesondere ihr Natriumsalz. Es ist besonders bevorzugt, den Gehalt an dem oder den Komplexbildnerkomponenten in der Desinfektionsmittelformulierung aus dem Bereich von 1 bis 10 Gew.-%, bezogen auf das Gesamtgewicht der Formulierung, zu wählen.

Erfindungsgemäß bevorzugte nichtionische Tenside sind schwachschäumende Fettalkoholpolyglykolether, z. B. lineare C12/C14-Alkoxylate mit Ethylen/Propylen/Buthylen-Copolymerisaten. Es ist besonders bevorzugt, den Gehalt an dem oder den nichtionischen Tensiden in der Desinfektionsmittelformulierung aus dem Bereich von 1 bis 10 Gew.-%, bezogen auf das Gesamtgewicht der Formulierung, zu wählen.

Vorteilhafter hydrophober Schaumregulator im Sinne der vorliegenden Erfindung ist N-Alkyl-Isononansäureamid. Es ist besonders bevorzugt, den Gehalt an dem oder den hydrophoben Schaumregulatoren in der Desinfektionsmittelformulierung aus dem Bereich von 0,1 bis 3 Gew.-%, bezogen auf das Gesamtgewicht der Formulierung, zu wählen.

Es ist erfindungsgemäß bevorzugt, die quaternäre(n) Ammoniumverbindung(en) aus der Gruppe Benzalkoniumchlorid, Dimethyldioctylammoniumchlorid, Didecyldimethylammoniumchlorid, Decylisononyldimethylammoniumchlorid und Didecylmethylpolyoxyethylammoniumpropionat zu wählen. Es ist ferner bevorzugt, den Gehalt an der oder den quatemären Ammoniumverbindung(en) in der Desinfektionsmittelformulierung aus dem Bereich von 1 bis 50 Gew.-%, vorzugsweise zwischen 1 und 10 Gew.-%, bezogen auf das Gesamtgewicht der Formulierung, zu wählen.

Sollen als Wirkstoff ein oder mehrere Alkylamine eingesetzt werden, so sind diese besonders vorteilhaft aus der Gruppe der Alkylamine zu wählen, welche sich durch die folgende Strukturformel auszeichnen: 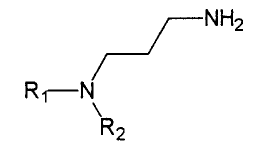 worin R₁ eine verzweigte oder unverzweigte, gesättigte oder ungesättigte Alkylgruppe mit 10 bis 16 Kohlenstoffatomen und R₂ eine Aminoalkylgruppe mit 1 bis 3 Kohlenstoffatomen, vorzugsweise (CH₂)₃NH₂, oder Wasserstoff darstellen. Vorteilhaft wird der Gehalt an dem oder den Alkylamin(en) in der Desinfektionsmittelformulierung aus dem Bereich von 0,1 bis 50 Gew.-%, vorzugsweise von 0,1 bis 5 Gew.-% gewählt, jeweils bezogen auf das Gesamtgewicht der Formulierung.

Erfindungsgemäß vorteilhafte Alkylthiouroniumsalze zeichnen sich durch folgende chemische Struktur aus 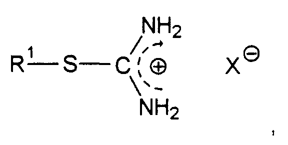 wobei R¹ gewählt wird aus der Gruppe der verzweigten und unverzweigten Alkylgruppen mit 1 bis 14 Kohlenstoffatomen, vorzugsweise mit 10 Kohlenstoffatomen, und X⁻ ein beliebiges Anion darstellt, vorzugsweise aber ein Halogenid-lon, wie z. B. Chlorid oder Bromid.

Bevorzugte α, ω-Alkylendithiouroniumsalze sind durch die folgende Struktur gekennzeichnet 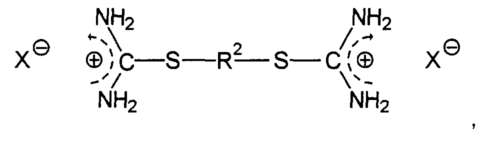 wobei R² gewählt wird aus der Gruppe der verzweigten und unverzweigten Alkylgruppen mit 1 bis 14 Kohlenstoffatomen, vorzugsweise mit 10 Kohlenstoffatomen, und X⁻ ein beliebiges Anion darstellt, vorzugsweise aber ein Halogenid-lon, wie z. B. Chlorid oder Bromid.

Vorteilhaft ist der Gehalt an einem oder mehreren Alkylthiouronium- und/oder α, ω-Alkylendithiouroniumsalzen in der Desinfektionsmittelformulierung zwischen 1 und 50 Gew.-%, vorzugsweise zwischen 1 und 10 Gew.-%, bezogen auf das Gesamtgewicht der Formulierung, zu wählen.

Die genannten Wirkstoffe können sowohl einzeln als auch in beliebigen Mischungen zueinander eingesetzt werden.

Erfindungsgemäß vorteilhaft wird der pH-Wert des Desinfektionsmittelkonzentrates auf Werte zwischen 6 bis 8 und vorzugsweise 6,5 bis 7,5 eingestellt. Hierdurch wird zum einen die Materialverträglichkeit der Anwendungslösung, zum anderen die Stabilität des Konzentrates verbessert. Zur pH-Einstellung können anorganische oder organische Säuren verwendet werden, wie beispielsweise das Sequestiermittel Hydroxyethan-1,1-diphosphonsäure oder andere Phosphonsäurederivate, welche zudem das Kalkbindevermögen der Anwendungslösung erhöht.

Es kann - auch wenn es nicht notwendig ist - von Vorteil sein, in erfindungsgemäßen Zubereitungen Lösungsvermittler einzusetzen, beispielsweise solche, die üblicherweise in Desinfektionsmitteln enthalten sind. Vorteilhafte Lösungsvermittler sind beispielsweise aliphatische Alkohole, wie beispielsweise n-Propanol, iso-Propanol, Butanol-2, Butanol-1, iso-Butanol und/oder Glykole mit 2 bis 5 Kohlenstoffatomen. Es wird bevorzugt, den Gehalt an einem oder mehreren Lösungsvermittlem zwischen 1 und 50 Gew.-%, insbesondere zwischen 1 und 20 Gew.-%, bezogen auf das Gesamtgewicht der Zubereitungen, zu wählen.

Ferner können die erfindungsgemäßen Zubereitungen vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 0,5 Gew.-%, bezogen auf das Gesamtgewicht der Zubereitungen, zu wählen.

Zusätzlich zu den vorstehend genannten Komponenten können die erfindungsgemäßen Zubereitungen für derartige Zubereitungen übliche Konservierungsstoffe, Farbstoffe, Duftstoffe und/oder andere übliche Hilfsstoffe, wie Komplexbildner und Schaumregulatoren, enthalten. Diese tragen in der Mehrzahl der Fälle nicht zur desinfizierenden Wirkung bei, sondern dienen der Lagerbarkeit sowie ästhetischen Zwecken. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindungen zu beschreiben, selbstverständlich ohne daß beabsichtigt ist, die Erfindungen auf diese Beispiele zu beschränken. Alle Prozentanteile sind, soweit nicht anders angegeben, auf das Gesamtgewicht der Zubereitungen bezogen.

### Beispiele:

| Angaben in Gew.-% | | |
|---|---|---|
| | | |
| Beispiel 1 | Monoethylenglykol | 3,0 % |
| | Dodecylbispropylentriamin | 1,0 % |
| | Dioctyldimethylammoniumchlorid | 6,0 % |
| | Benzalkoniumchlorid | 1,5 % |
| | Fettalkoholpolyglykolether | 4,0 % |
| | Entschäumer (Hauptbestandteil: N-Alkyl-lsononansäureamid) | 2,3 % |
| | Nitrilotriessigsäure, Na-Salz | 5,6 % |
| | Hydroxyethan-1,1-diphosphonsäure | 1,8 % |
| | rrosionsinhibitoren | 1,0 % |
| | Rest: Wasser | |
| | | |
| Beispiel 2 | Monoethylenglykol | 15,0 % |
| | Dodecylbispropylentriamin | 1,0 % |
| | Dioctyldimethylammoniumchlorid | 4,0 % |
| | Benzalkoniumchlorid | 0,75 % |
| | Decylthiouroniumchlorid | 2,4 |
| | Fettalkoholpolyglykolether | 5,0 % |
| | Entschäumer (Hauptbestandteil: N-Alkyl-lsononansäureamid) | 2,3 % |
| | Nitrilotriessigsäure, Na-Salz | 5,6 % |
| | Hydroxyethan-1,1-diphosphonsäure | 1,8 % |
| | rrosionsinhibitoren | 1,2 % |
| | Rest: Wasser | |

Beide Rezepturen sind stabil und bilden in wässriger Verdünnung eine schaumarme Emulsion.

## Patentansprüche

1. Desinfektionsmittelformulierungen, welche neben einem Gehalt an Wirkstoffen gewählt aus den Gruppen quaternäre Ammoniumverbindungen, Alkylamine, Alkylthiouroniumsalze, α, ω-Alkylendithiouroniumsalze und/oder Gemische derselben
a) 1 bis 50 Gew.-% mindestens einer Komplexbildnerkomponente,
b) 1 bis 50 Gew.-% mindestens eines nichtionischen Tensids und
c) 0,1 bis 10 Gew.-% mindestens eines hydrophoben Schaumregulators enthalten,
wobei die Prozentangaben jeweils auf das Gesamtgewicht der Formulierungen bezogen sind.

2. Desinfektionsmittelformulierung nach Anspruch 1, **dadurch gekennzeichnet, daß** zusätzlich Tenside und/oder Hilfs-, Zusatz- und/oder Wirkstoffe enthalten sind.

3. Desinfektionsmittelformulierung nach einem der vorhergehenden Ansprüche, **dadurch gekennzeichnet, daß** es sich um Konzentrate handelt, die vor der Verwendung mit Wasser zu einer Gebrauchslösung verdünnt werden.

4. Desinfektionsmittelformulierung nach einem der vorhergehenden Ansprüche, **dadurch gekennzeichnet, daß** die Komplexbildnerkomponente(n) aus der Gruppe Nitriloessigsäure und ihre Salze gewählt werden.

5. Desinfektionsmittelformulierung nach einem der vorhergehenden Ansprüche, **dadurch gekennzeichnet, daß** das oder die nichtionischen Tenside aus der Gruppe der schwachschäumenden Fettalkoholpolyglykolether gewählt werden.

6. Desinfektionsmittelformulierung nach einem der vorhergehenden Ansprüche, **dadurch gekennzeichnet, daß** als hydrophober Schaumregulator N-Alkyl-lsononansäureamid gewählt wird.

7. Desinfektionsmittelformulierung nach einem der vorhergehenden Ansprüche, **dadurch gekennzeichnet, daß** der Gehalt an einem oder mehreren Wirkstoffen aus dem Bereich von 0,1 bis 50 Gew.-% gewählt wird, bezogen auf das Gesamtgewicht der Zubereitungen.

8. Schwachschäumende Desinfektionsmittelformulierung, welche als Konzentrat vorliegt, **dadurch gekennzeichnet, daß** einerseits das Konzentrat in einem Temperaturbereich von 10 bis 50 °C eine klare einphasige Mischung darstellt und daß andererseits dessen wässrige Verdünnung, welche 0,2 bis 2,0 Gew.-% an Konzentrat enthält, in einem Temperaturbereich von 20 bis 60° C eine trübe, mehrphasige Emulsion darstellt.

9. Verwendung von Gebrauchslösungen nach Anspruch 3 als Desinfektionsmittel für medizinisch-chirurgische Instrumente in Wasch- und/oder Desinfektionsautomaten.

10. Verwendung von Gebrauchslösungen nach Anspruch 3 als Desinfektionsmittel für die chemo-thermische Aufbereitung flexibler Endoskope.
